# FASCICULE DE BREVET EUROPEEN

(11) **EP 2 468 217 B1**
(45) Date de publication et mention de la délivrance du brevet: **16.03.2016**
(21) Numéro de dépôt: 11194904.6
(22) Date de dépôt: 21.12.2011
(51) Int. Cl.: A61F 2/42, A61F 2/30

(54) **Implant d'hémi-arthroplastie métatarsien**
Hemiarthroplastik-Implantat für Mittelfußknochen
Metatarsal hemiarthroplasty implant

(30) Priorité: 29.03.2011 FR 1152587; 21.12.2010 US 201061425745 P
(43) Date de publication de la demande: 27.06.2012
(73) Titulaire: Tornier, 38330 Montbonnot-Saint-Martin (FR)
(72) Inventeur: Ol, Nelson, San Diego, CA 92122 (US); Wilson Wirth, Corey, San Diego, CA 92101 (US); Hassler, Michel, 38330 Saint Ismier (FR); Vancelette, David, San Diego, CA 92126 (US); Weil, Lowell Scott, Glenview, IL 60025 (US); Deland, Jonathan, New York, NY 10021 (US); Harber, Chris, Mound, MN 55364 (US); Burstein, Albert H., Reno, NV 89510 (US)
(74) Mandataire: Grand, Guillaume

(56) Documents cités:
- EP-A1- 1 508 316
- EP-A2- 1 637 095
- DE-A1- 10 130 796
- FR-A1- 2 465 470
- FR-A1- 2 709 948

## Description

La présente invention concerne un implant d'hémi-arthroplastie métatarsien.

L'invention s'intéresse ainsi aux implants destinés à être mis en place à l'articulation entre un métatarsien, en particulier le premier métatarsien, et sa phalange associée, du pied d'un être humain. L'invention s'intéresse plus particulièrement au cas où, au niveau de l'articulation métatarso-phalangienne, la tête d'articulation du métatarsien est affectée, typiquement par un hallux rigidus ou arthrose rigidifiante, ce qui provoque une gêne et des douleurs pour le patient.

Une possibilité de traitement chirurgical, non concernée par l'invention, consiste à fusionner l'articulation métatarso-phalangienne, ce qui induit sa perte irrémédiable de mobilité.

Une autre approche de traitement, non concernée par l'invention, consiste à implanter, uniquement sur la phalange, un composant prothétique métallique.

Encore une autre approche de traitement, également non concernée par l'invention, consiste à poser, au niveau de l'articulation métatarso-phalangienne, une prothèse articulaire totale, incluant un implant métatarsien et un implant phalangien, articulés l'un contre l'autre. Ainsi, DE-A-101 30 796, EP-A-1 508 316 et FR-A-2 709 948 proposent des prothèses métatarso-phalangiennes totales dont l'implantation nécessite de réséquer grandement les extrémités osseuses du métatarsien et de la phalange à prothéser : en particulier, du côté du métatarsien, sa tête articulaire est réséquée quasiment en totalité de manière à délimiter plusieurs plans de coupe successifs contre lesquels des surfaces planes respectives, délimitées par la face de fixation de l'implant, sont plaquées lors de la pose de cet implant. De son côté, FR-A-2 465 470 propose une prothèse métatarso-phalangienne totale dont l'implantation nécessite de retirer en totalité la tête du métatarsien pour la remplacer par deux calottes sphériques adjacentes, ce qui procure une géométrie d'articulation très éloignée de l'anatomie.

Une approche chirurgicale alternative, relevant du domaine de l'invention, repose sur la mise en place, uniquement sur la tête du métatarsien, d'un implant articulaire, qualifié ainsi d'implant d'hémi-arthroplastie puisque, après implantation, cet implant s'articule directement contre la phalange, non prothésée. Actuellement, on connaît et on dispose sur le marché d'implants d'hémi-arthroplastie métatarsiens relativement massifs, dont le corps métallique est implanté en lieu et place de la tête articulaire anatomique du métatarsien, qui, à cette fin, est soit réséquée en totalité, soit significativement entamée. L'ancrage de ce corps métallique est réalisé par une quille centrale, qui présente des dimensions longitudinale et transversale importantes pour retenir mécaniquement l'implant après avoir enfoncé profondément cette quille dans le métatarsien. La mise en place de cet implant implique donc le sacrifice d'un volume osseux important du métatarsien, ce qui fragilise l'os et complique une éventuelle intervention ultérieure de fusion de l'articulation. De plus, dans la mesure où le prothésage de la tête du métatarsien est typiquement centré sur l'axe longitudinal central de ce métatarsien, on constate souvent que, d'une part, l'implant interfère avec la plaque plantaire lors des mouvements de la marche du patient, ce qui induit une forte gêne pour le patient, voire endommage les sésamoïdes, et, d'autre part, l'implantation réalisée ne traite pas d'éventuels ostéophytes dorsaux du métatarsien, qui sont pourtant particulièrement douloureux pour le patient. EP 1637095 A2 (voir le paragraphe 40) propose un implant d'hémi-asthroplastic métatarsien qui s'articule directement contre la phalange, non prothésée.

Le but de la présente invention est de proposer un implant d'hémi-arthroplastie, qui, tout en étant facile à implanter, soit plus performant en service.

A cet effet, l'invention a pour objet un implant d'hémi-arthroplastie métatarsien, tel que défini à la revendication 1.

Une des idées à la base de l'invention est de chercher à limiter la quantité de matière osseuse retirée du métatarsien pour les besoins de la mise en place et de la fixation de l'implant, tout en optimisant le positionnement de cet implant sur la tête du métatarsien. Ainsi, selon l'invention, le corps de l'implant est conçu pour resurfacer principalement, voire exclusivement la face distale et la face dorsale de la tête du métatarsien. La face plantaire de la tête du métatarsien n'est alors ni prothésée, ni même entamée lors de la préparation osseuse de cette tête, ce qui préserve la plaque plantaire et les sésamoïdes et évite toute interférence de l'implant avec ces derniers, y compris lors de la dorsiflexion complète du pied du patient. De plus, la face de l'implant, qui va être fixée sur la tête du métatarsien, en couvrant les zones distale et dorsale préalablement préparées de cette tête, est prévue concave pour conserver au maximum la matière osseuse présente, tout en étant dépourvue d'une tige centrale d'ancrage massive ou d'un unique élément massif similaire, au profit d'au moins deux éléments saillants distincts, qui s'étendent ainsi en saillie depuis une même surface concave de l'implant et qui sont, comparativement à un unique élément massif, moins étendus en profondeur dans le métatarsien, mais qui, grâce à leur nombre et/ou à leur disposition relative, assurent un blocage mécanique efficace de l'implant, notamment en rotation et en cisaillement vis-à-vis du métatarsien. Le stock de matière osseuse du métatarsien est ainsi préservé au mieux, en évitant de fragiliser en profondeur le métatarsien et en laissant la possibilité de fusionner ultérieurement l'articulation métatarso-phalangienne, sans pour autant nuire à la stabilité mécanique de l'implant conforme à l'invention. Sur sa face opposée à celle fixée au métatarsien, le corps de l'implant selon l'invention est conformé de manière convexe, avantageusement de façon complémentaire à la surface articulaire native de la phalange non prothésée, afin de coopérer au mieux avec cette dernière pour reproduire la cinématique naturelle d'origine de l'articulation métatarso-phalangienne. Pour restaurer de manière aussi fidèle et pérenne que possible l'interface articulaire entre le métatarsien prothésé et la phalange native, la surface articulaire convexe de l'implant est, selon l'invention, réalisée en céramique, notamment en pyrocarbone, c'est-à-dire que cette surface est délimitée par une partie du corps de l'implant, constituée de la céramique précitée. L'usure de la phalange s'en trouve significativement limitée, voire rendue quasinulle.

Des caractéristiques additionnelles avantageuses de l'implant conforme à l'invention, prises isolément ou selon toutes les combinaisons techniquement possibles, sont spécifiées aux revendications 2 à 12.

L'invention sera mieux comprise à la lecture de la description qui va suivre, donnée uniquement à titre d'exemple et faite en se référant aux dessins sur lesquels :
- les figures 1 et 2 sont des vues en perspective, sous des angles de vue différents, d'un implant conforme à l'invention ;
- la figure 3 est une vue en élévation selon la flèche III de la figure 1 ;
- les figures 4 et 5 sont des coupes respectivement selon les lignes IV-IV et V-V de la figure 3 ;
- la figure 6 est une vue dorsale d'une articulation métatarso-phalangienne, en présence de l'implant des figures précédentes ; et
- la figure 7 est une coupe dans le plan de la figure 4, illustrant l'implant en place au sein de l'articulation de la figure 6.

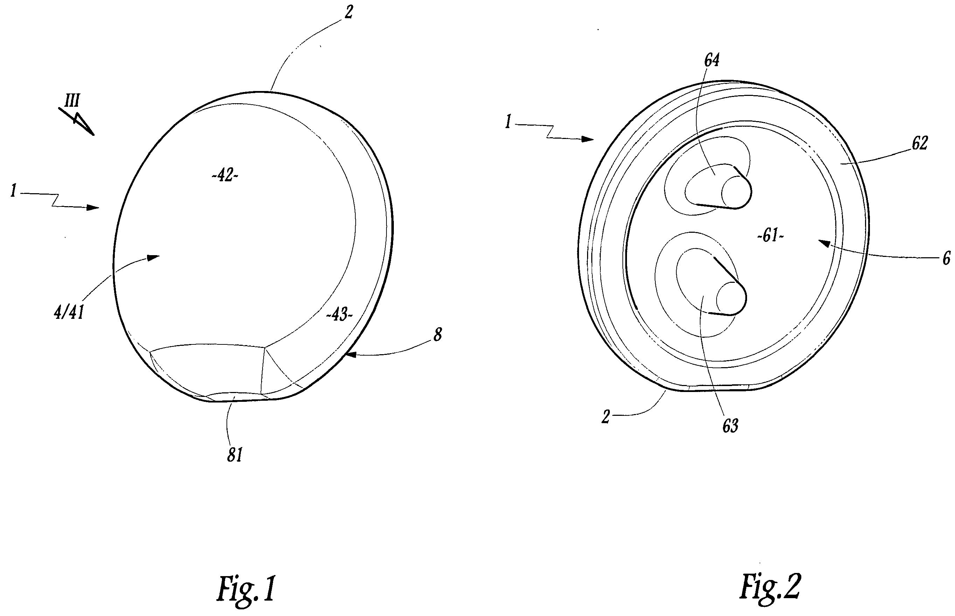

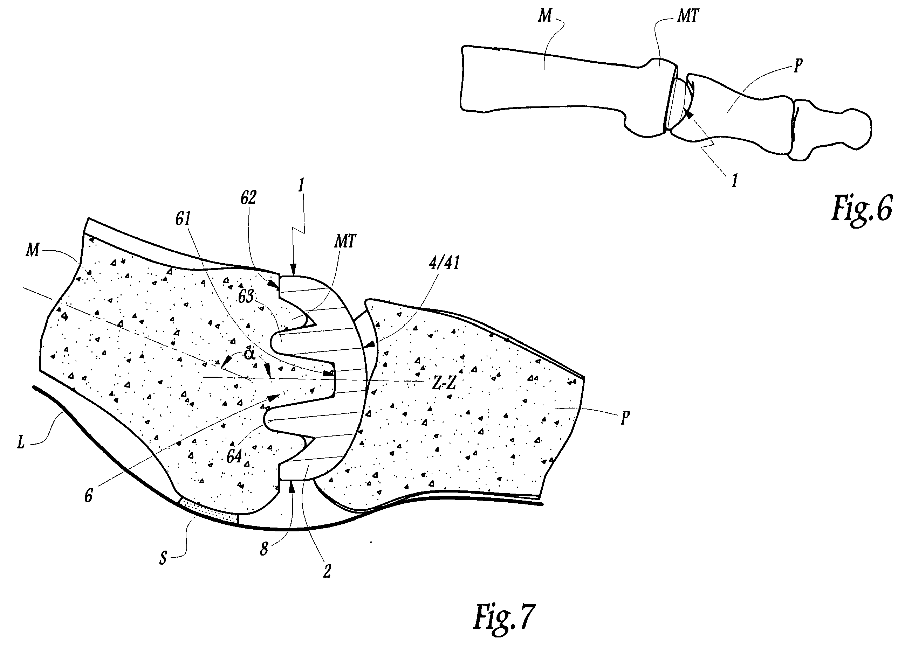

Sur les figures 1 à 5 est représenté un implant d'hémi-arthroplastie métatarsien 1 adapté pour être implanté entre un métatarsien M, en particulier le premier métatarsien, et la phalange P associée à ce métatarsien, comme représenté sur les figures 6 et 7, en vue de rétablir l'articulation métatarso-phalangienne entre les os précités du pied d'un patient.

Dans tout le présent document, lorsqu'un terme ayant un sens anatomique usuel, tel que le mot « sagittal » par exemple, est utilisé pour décrire l'implant 1, l'implant est considéré dans sa configuration d'implantation au sein de l'articulation métatarso-phalangienne.

L'implant 1 comporte un corps 2, qui va être décrit en détail ci-après et qui, globalement, présente la forme d'une portion de boule creuse. Le corps 2 présente ainsi deux faces principales opposées 4 et 6, qui sont séparées l'une de l'autre par l'épaisseur du corps 2 et qui, dans l'exemple de réalisation considéré sur les figures, ne se raccordent pas directement l'une à l'autre, mais sont reliées l'une à l'autre par une surface périphérique 8 du corps 2, qui est cylindrique et dont la génératrice est parallèle à un axe géométrique central référencé Z-Z. Dans la configuration d'implantation de l'implant 1, les faces 4 et 6 sont respectivement tournées vers la phalange P et vers le métatarsien M.

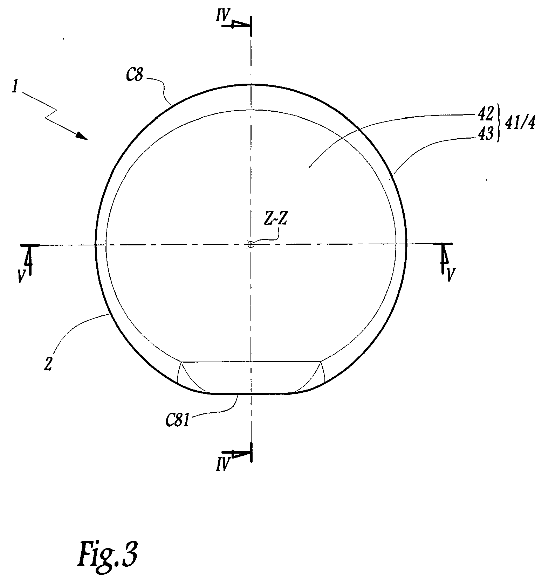

Dans le mode de réalisation considéré sur les figures, la surface périphérique 8 du corps 2 est cylindrique à base sensiblement circulaire, centrée sur l'axe Z-Z, comme bien visible sur les figures 3 à 5. La base du cylindre précitée n'est pas rigoureusement circulaire, dans le sens où cette base est effectivement circulaire sur toute la périphérie du corps 2, c'est-à-dire qu'elle présente un rayon constant depuis l'axe Z-Z, excepté du côté plantaire du corps 2, c'est-à-dire sur une portion de ce corps, qui, en service, est tournée vers la plante du pied auquel appartiennent le métatarsien M et la phalange P. Comme bien visible sur la figure 3, le contour circulaire C8 que présente le corps 2 en projection dans un plan perpendiculaire à l'axe Z-Z est ainsi interrompu par une corde C81 qui s'étend, suivant une direction médio-latérale, du côté plantaire de ce contour. En volume, cela revient à ce que la surface périphérique cylindrique 8 présente, du côté plantaire du corps 2, une portion plate 81, bien visible sur la figure 1. Un premier intérêt de cet agencement est de fournir un repère de détrompage à l'attention du chirurgien, dans le sens où, lors de la mise en place de l'implant 1, le chirurgien dispose ainsi d'une caractéristique visuelle lui indiquant le côté du corps 2 destiné à être tourné vers la plante du pied. Un autre intérêt de cet agencement apparaîtra plus loin.

Comme évoqué plus haut, la face 4 du corps 2 s'apparente, globalement, à une portion de sphère. Plus précisément, sur cette face 4, le corps 2 délimite une surface convexe 41 qui, dans le mode de réalisation représenté sur les figures, occupe la totalité de la face 4.

En service, la surface convexe 41 est destinée à s'articuler directement contre la phalange P. A cette fin, la surface convexe 41 est réalisée en céramique, typiquement en pyrocarbone, autrement appelé carbone pyrolytique. Autrement dit, au moins dans sa partie délimitant la surface 41, le corps 2 est réalisé en la céramique précitée. D'ailleurs, dans l'exemple de réalisation considéré sur les figures, le corps 2 est avantageusement réalisé d'un seul tenant, en un bloc de céramique. Cela confère à la surface 41 une bonne biocompatibilité, une haute résistance mécanique et une élasticité proche de l'os, tout en limitant, voire quasi annulant l'usure de la phalange P.

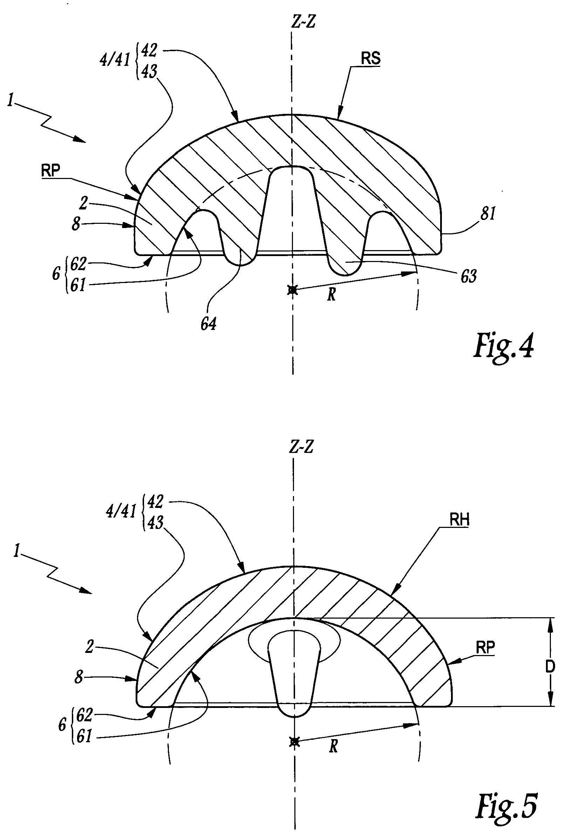

Comme expliqué en détail ci-après, cette surface convexe 41 est avantageusement à courbure non constante. Plus précisément, dans la région centrale 42 de cette surface convexe 41, c'est-à-dire la région qui, dans l'exemple de réalisation considéré ici, est globalement centrée sur l'axe Z-Z, la surface 41 présente :
- dans le plan de la figure 4, autrement dit en coupe dans un plan médian sagittal du corps 2, un rayon de courbure noté RS, et
- dans le plan de la figure 5, c'est-à-dire en coupe dans un plan médian horizontal du corps 2, un rayon de courbure qui est noté RH et qui est strictement supérieur au rayon de courbure RS.

De cette façon, la surface convexe 41 présente, dans sa région centrale 42, une géométrie typiquement complémentaire de la surface articulaire anatomique de la phalange P, de sorte que, dans la configuration d'implantation de l'implant 1, la surface convexe 41 s'articule contre la phalange P avec une cinématique similaire, voire quasi identique à la cinématique naturelle d'une articulation métatarso-phalangienne saine, non prothésée.

Dans le même esprit, la région périphérique 43 de la surface convexe 41, c'est-à-dire la région de cette surface raccordant la région centrale 42 à la surface périphérique 8 du corps 2, suivant toute la périphérie de la face 4, ne présente pas la même courbure que la région centrale 42, mais un rayon de courbure noté RP sur les figures 4 et 5. Ce rayon de courbure RP est constant suivant toute la périphérie de la région 43, excepté du côté plantaire du corps 2, en raison du raccordement de la région centrale 42 à la portion plate 81 de la surface périphérique 8. En pratique, le rayon de courbure RP de la région périphérique 43 est strictement inférieur au rayon de courbure RS de la région centrale 42, notamment en valant moins de la moitié de ce rayon de courbure RS.

A titre d'exemple numérique non limitatif, eu égard aux dimensions caractéristiques de l'articulation métatarso-phalangienne d'un patient adulte, le rayon de courbure RS est par exemple choisi entre 15 et 30 mm, le rayon de courbure RH présentant une valeur augmentée d'environ 2 mm par rapport à celle du rayon de courbure RS, tandis que le rayon de courbure RP présente une valeur comprise entre 3 et 5 mm.

La face 6 du corps 2 inclut une surface concave 61 qui n'occupe pas toute cette face 6, mais qui occupe uniquement la région centrale de cette face 6, en étant entourée par une bordure 62 délimitée par le corps 2 tout autour de la surface concave 61.

Dans le mode de réalisation représenté sur les figures, la surface concave 61 est de nature sphérique, cette surface 61 correspondant ainsi à une calotte d'une sphère géométrique indiquée partiellement en traits mixtes sur les figures 4 et 5. Avantageusement, dans le mode de réalisation considéré sur les figures, le plan géométrique, qui, par intersection de la sphère géométrique précitée, détermine la calotte 61, est perpendiculaire à l'axe Z-Z et correspond sensiblement au plan contenant la bordure 62. Ainsi, la calotte 61 est centrée sur l'axe Z-Z, dans le sens où cet axe constitue un axe de révolution pour la calotte. Le rayon de la calotte 61 est noté R sur les figures 4 et 5 : ce rayon R est strictement inférieur au rayon de courbure RS de la surface convexe 41. En particulier, dans le cadre de l'exemple numérique évoqué plus haut, cette valeur R est notamment plus petite d'au moins 2 mm que la valeur du rayon de courbure RS.

On notera que, par définition, la calotte 61 correspond à moins de la moitié d'une demi-sphère : autrement dit, le plan définissant la calotte 61, par intersection de la sphère géométrique indiquée en traits mixtes sur les figures, ne passe pas par le centre de cette sphère. Avantageusement, pour des raisons détaillées plus loin, visant à limiter l'étendue de la calotte 61, la profondeur D de cette calotte, c'est-à-dire la distance suivant l'axe Z-Z entre le plan précité et la surface 61, est inférieure à 75% du rayon R.

De plus, comme bien visible sur les figures 2, 4 et 5, le corps 2 est pourvu, sur sa face 6, de deux éléments distincts 63 et 64 qui s'étendent en saillie depuis la surface concave 61. Dans l'exemple de réalisation considéré sur les figures, les éléments 63 et 64 s'étendent suivant des directions de saillie respectives qui sont sensiblement parallèles à l'axe Z-Z. Ces éléments 63 et 64 s'étendent ainsi depuis la surface 61 sur des dimensions saillantes respectives telles que les extrémités libres de ces éléments sont situées du côté du plan contenant la bordure 62, opposé à la surface 61. Pour des raisons qui apparaîtront plus loin, la dimension saillante de l'élément 63, c'est-à-dire celui des deux éléments le plus proche du côté plantaire du corps 2, est strictement supérieure à la dimension saillante de l'élément 64. Par ailleurs, comme bien visible sur la figure 4, les éléments 63 et 64 sont répartis dans un plan médian sagittal du corps 2, et ce de manière sensiblement régulière, dans le sens où, dans le plan médian sagittal précité, la distance séparant l'un de l'autre les axes respectifs des éléments 63 et 64 est sensiblement égale à la distance entre chacun de ces axes et la portion la plus proche de la surface périphérique 8 du corps 2.

En pratique, on notera que la forme individuelle des éléments 63 et 64 n'est pas limitative de la présente invention. Ainsi, dans l'exemple de réalisation considéré sur les figures, chaque élément 63, 64 présente une forme globalement tronconique, qui est centrée sur un axe parallèle à l'axe Z-Z et divergente en direction de la surface concave 61. Toutefois, d'autres formes géométriques sont tout aussi bien envisageables, par exemple des formes cylindriques à base circulaire, elliptique ou polygonale.

L'intérêt de l'agencement de la face 6 du corps 2 de l'implant 1, notamment avec sa calotte 61 et ses éléments 63, 64, va être mieux compris ci-dessous, dans le cadre de la description d'un exemple d'une intervention chirurgicale visant à mettre en place l'implant 1 au niveau de l'articulation entre le métatarsien M et la phalange P.

Dans un premier temps opératoire, un chirurgien accède à la zone articulaire entre le métatarsien M et la phalange P, notamment via une voie d'abord dorsale. Après avoir écarté l'un de l'autre le métatarsien M et la phalange P afin d'élargir l'espace interosseux, le chirurgien prépare la tête MT du métatarsien de manière à la conformer de façon adaptée à la face 6 du corps 2. Pour ce faire, le chirurgien utilise par exemple une fraise qu'il va appliquer sur les faces dorsale et distale de la tête métatarsienne, pour retirer la couche de surface de cette tête, constituée de cartilage et de matière osseuse. L'épaisseur de matière cartilo-osseuse ainsi retirée est de l'ordre de 2 à 5 mm uniquement, ce qui est suffisant pour retirer les parties altérées du métatarsien, aussi bien sur son côté distal qui, jusqu'alors s'articulait avec la phalange P, que sur son côté dorsal, qui pouvait notamment être le lieu d'ostéophytes d'origine arthritique. Avantageusement, l'opération de préparation de la tête MT du métatarsien M, notamment l'application de la fraiseuse précitée, est guidée par une broche préalablement implantée dans le métatarsien M. A l'issue de cette opération de préparation de la tête MT du métatarsien M, cette dernière présente un état de surface complémentaire à la surface concave 61 et à la bordure 62 de la face postérieure 6 du corps 2.

Dans un second temps opératoire, le chirurgien met en place l'implant 1 sur la tête métatarsienne MT, en venant coiffer la surface préparée de cette tête par le corps 2. En rapprochant la face 6 du corps 2 vis-à-vis de la surface préparée de la tête MT du métatarsien M, les éléments 63 et 64 pénètrent dans la matière osseuse du métatarsien M. Le cas échéant, pour faciliter cette pénétration, des pré-trous peuvent être préalablement réalisés dans la surface préparée de la tête métatarsienne. Par ailleurs, comme les éléments 63 et 64 s'étendent parallèlement les uns aux autres, leur insertion dans le métatarsien s'effectue avantageusement sous l'application d'un effort dirigé selon la direction de parallélisme des éléments, à savoir selon l'axe Z-Z pour le mode de réalisation représenté sur les figures, ce qui facilite l'engagement mécanique de ces éléments dans la matière osseuse constituant le métatarsien. En particulier, la pénétration des éléments 63 et 64 dans cette matière osseuse est réalisée par une impaction appliquée sur la face 4 du corps 2.

Dans tous les cas, dès lors que la surface 61 se trouve appuyée contre la surface préparée complémentaire de la tête MT du métatarsien M, les éléments 63 et 64 s'étendent dans la matière osseuse du métatarsien, immobilisant ainsi mécaniquement le corps 2 vis-à-vis du métatarsien, notamment en rotation autour de l'axe Z-Z et en cisaillement transversalement à cet axe. Dans le même temps, la bordure 62 s'appuie contre une portion complémentaire dédiée de la surface préparée de la tête MT du métatarsien M, comme représenté sur la figure 7, notamment à des fins de transmission de contraintes suivant l'axe Z-Z entre l'implant 1 et le métatarsien M.

Une fois que l'implant 1 est ainsi positionné sur le métatarsien M, l'écartement imposé entre le métatarsien et la phalange P est relâchée de sorte que, sous l'action des ligaments, éventuellement reconstruits par le chirurgien, entourant l'articulation, la phalange vient s'appuyer et coopérer de manière articulaire contre la surface convexe 41 du corps 2 de l'implant 1. Le chirurgien peut ensuite refermer les tissus mous autour de l'articulation métatarso-phalangienne.

Ainsi, on comprend que le corps 2 de l'implant 1 permet de réaliser un resurfaçage distal et dorsal de la tête MT du métatarsien M. Cela est lié au fait que le corps ne présente qu'une faible épaisseur, c'est-à-dire une faible distance séparant l'une de l'autre les surfaces convexe 41 et concave 61, qui correspond à l'épaisseur de cartilage et d'os qui a été retirée de la tête du métatarsien, lors de la préparation de cette tête. Eu égard à cette faible épaisseur du corps 2, typiquement de l'ordre de 2 à 5 mm dans le cadre de l'exemple numérique évoqué plus haut, le positionnement dorsal et distal du corps 2 sur la tête MT du métatarsien M, visant à resurfacer cette tête, permet de préserver le côté plantaire de la tête métatarsienne, en particulier en limitant, voire en évitant toute interférence de l'implant 1 avec les sésamoïdes S et la plaque plantaire L, comme représenté sur la figure 7, y compris lors des mouvements de marche du pied, notamment lors d'une dorsiflexion complète du pied.

Dans ce contexte de préservation des sésamoïdes S et de la plaque plantaire L, on comprend qu'il est préférable que l'étendue de la face postérieure 6 du corps 2 soit limitée : en limitant la profondeur D de la calotte 61 à 75% du rayon R, comme cela a été spécifié plus haut, on limite l'étendue de la tête métatarsienne MT qui se retrouve couverte par le corps de resurfaçage 2, cette étendue correspondant par exemple uniquement aux trois quarts supérieurs de la tête métatarsienne MT, voire moins.

Avantageusement, toujours dans la contexte de préservation des sésamoïdes S et de la plaque plantaire L et de l'optimisation de l'étendue de couverture de la tête MT du métatarsien M par le corps 2, l'implant 1 est mis en place de manière que son axe Z-Z soit incliné à environ 165° de l'axe central du métatarsien M, comme indiqué par l'angle référence α sur la figure 7.

De plus, la limitation, voire l'absence d'interférence entre le corps 2 et les sésamoïdes S et la plaque plantaire L est favorisée par la portion plate 81 de la surface périphérique 8 du corps 2, eu égard au fait que cette portion plate est située du côté plantaire du corps 2.

Divers aménagements et variantes à l'implant 1 et à sa méthode d'implantation sont par ailleurs envisageables, comme évoqué ci-après à titre d'exemples.

En particulier, le nombre et/ou l'agencement des éléments saillants prévus sur la face postérieure 6 de l'implant 1, tels que les éléments 63 et 64 dans le mode de réalisation montré sur les figures, peuvent être modifiés. Ainsi, trois ou quatre, voire davantage d'éléments saillants de ce type peuvent être prévus, étant remarqué qu'au moins deux tels éléments sont nécessaires pour garantir une immobilisation mécanique satisfaisante, tout en limitant la dimension saillante de ces éléments, notamment à des fins de préservation de la matière osseuse du métatarsien M. Ainsi, selon une variante non représentée, plutôt que les deux éléments 63 et 64 soient disposés dans un plan médian sagittal du corps 2, deux éléments similaires peuvent être disposés dans un autre plan, passant par l'axe Z-Z, notamment dans le plan médian horizontal du corps 2. Dans ce cas, ces deux éléments sont avantageusement prévus sensiblement symétriques l'un de l'autre par rapport au plan médian sagittal du corps 2, afin d'équilibrer les contraintes mécaniques d'immobilisation de l'implant sur le métatarsien M. Par ailleurs, dans tous les cas, le ou les éléments les plus près du côté plantaire du corps 2, c'est-à-dire celui ou ceux qui, suivant une direction dorso-plantaire, sont situés le plus proche du côté plantaire du corps 2, présentent une dimension saillante la plus grande par rapport aux autres éléments saillants présents : en effet, ce ou ces éléments les plus proches du côté plantaire, à l'exemple de l'élément 63 pour l'implant 1 des figures 1 à 7, sont en effet engagés dans la zone a priori la plus épaisse de la tête MT du métatarsien M, ce qui permet ainsi de bénéficier d'une meilleure stabilisation mécanique de l'implant 1 sur cette tête, notamment d'un grand bras de levier pour résister aux contraintes lors d'une dorsiflexion de l'orteil. De surcroît, ce ou ces éléments plus longs fournissent au chirurgien un repère d'orientation supplémentaire pour faciliter la mise en place de l'implant.

A la différence du mode de réalisation montré sur les figures 1 à 7, dans lequel le corps 2 est réalisé d'une seule pièce en céramique y compris les éléments 63 et 64, tout ou partie de la face 6 peut être réalisée en un autre matériau. En particulier, les éléments saillants, tels que les éléments 63 et 64, peuvent être au moins partiellement constitués d'un matériau favorisant l'ostéo-intégration, visant à renforcer la fixation mécanique de ces éléments dans la matière osseuse constituant le métatarsien M. De plus, indépendamment de la nature du matériau utilisé pour réaliser la face 6 du corps 2, une autre variante, non représentée, consiste à prévoir que, plutôt que d'être venus de matière avec la surface 61, les éléments 63 et 64 peuvent être rapportés, le cas échéant de manière amovible, sur la surface concave 61 : dans ce cas, une pluralité d'emplacements d'assemblage, le cas échéant amovible, est prévue sur cette surface concave 61 et, avant l'implantation de l'implant 1, le chirurgien assemble au moins deux éléments saillants au corps 2, en les fixant, par exemple par vissage ou par emmanchement, dans des emplacements respectifs qu'il aura sélectionnés parmi la pluralité d'emplacements à disposition.

Dans tous les cas, en prévoyant que l'implant 1, dans sa globalité, présente comme plan de symétrie son plan médian sagittal, l'implant aura l'avantage de pouvoir être fixé indifféremment sur un métatarsien gauche et sur un métatarsien droit d'un patient.

## Revendications

1. Implant d'hémi-arthroplastie métatarsien (1),
comportant un corps (2) de resurfaçage distal et dorsal de la tête articulaire (MT) d'un métatarsien (M), lequel corps délimite :
- sur sa face (4) destinée à s'articuler contre la phalange (P) associée au métatarsien, une surface convexe (41) qui est réalisée en céramique, notamment en pyrocarbone, et
- sur sa face (6) destinée à se fixer au métatarsien (M), une surface concave (61) depuis laquelle s'étendent en saillie au moins deux éléments (63, 64) d'immobilisation du corps (2) sur la tête (MT) du métatarsien.

2. Implant suivant la revendication 1, **caractérisé en ce que** les éléments d'immobilisation (63, 64) s'étendent en saillie de la surface concave (61) suivant des directions respectives qui sont sensiblement parallèles.

3. Implant suivant l'une des revendications 1 ou 2, **caractérisé en ce que** le (63) ou les éléments d'immobilisation, qui, suivant une direction dorso-plantaire, sont le plus proche du côté plantaire du corps (2), présentent une dimension saillante la plus grande.

4. Implant suivant l'une quelconque des revendications précédentes, **caractérisé en ce que** les deux (63, 64) ou au moins deux des éléments d'immobilisation sont répartis de manière distincte dans un plan médian sagittal du corps (2).

5. Implant suivant l'une quelconque des revendications précédentes, **caractérisé en ce que** les deux ou au moins deux des éléments d'immobilisation sont sensiblement symétriques l'un de l'autre par rapport à un plan médian sagittal du corps (2).

6. Implant suivant l'une quelconque des revendications précédentes, **caractérisé en ce que** la surface concave (61) comprend, voire consiste en une calotte sensiblement sphérique.

7. Implant suivant la revendication 6, **caractérisé en ce que** la profondeur (D) de la calotte (61) est inférieure à 75% du rayon (R) de la sphère géométrique à laquelle la calotte appartient.

8. Implant suivant l'une quelconque des revendications précédentes, **caractérisé en ce que** le corps (2) délimite, sur sa face (6) destinée à se fixer au métatarsien (M), une bordure d'appui osseux (62) qui court autour de la surface concave (61), le cas échéant en étant disposé sensiblement dans le plan géométrique définissant la calotte.

9. Implant suivant l'une quelconque des revendications précédentes, **caractérisé en ce que** la surface convexe (41) inclut une région centrale (42) qui, en coupe dans un plan médian sagittal du corps (2), présente un rayon de courbure (RS) inférieur à son rayon de courbure (RH) en coupe dans un plan médian horizontal du corps.

10. Implant suivant l'une quelconque des revendications précédentes, **caractérisé en ce que** le corps (2) délimite une surface périphérique (8) reliant ses deux faces (4, 6), qui présente un contour sensiblement circulaire (C8) interrompu par une corde (C81) au moins du côté plantaire du corps.

11. Implant suivant l'une quelconque des revendications précédentes, **caractérisé en ce que** le corps (2) est réalisé d'une seule pièce en céramique, notamment en pyrocarbone.

12. Implant suivant l'une quelconque des revendications 1 à 10, **caractérisé en ce que** les éléments d'immobilisation (63, 64) sont au moins partiellement constitués d'un matériau d'ostéo-intégration.

## Patentansprüche

1. Hemiarthroplastik-Implantat für Mittelfußknochen (1),
das einen Körper (2) zur distalen und dorsalen Oberflächenwiederherstellung des Gelenkkopfs (MT) eines Mittelfußknochens (M) umfasst, wobei der Körper Folgendes begrenzt:
- auf seiner Fläche (4), die zur gelenkigen Lagerung gegen den Phalanx (P), der dem Mittelfußknochen zugehörig ist, bestimmt ist, eine konvexe Fläche (41), die aus Keramik hergestellt ist, insbesondere aus Pyrokohlenstoff, und
- auf seiner Fläche (6), die zur Befestigung an dem Mittelfußknochen (M) bestimmt ist, eine konkave Fläche (61), von der aus vorspringend sich mindestens zwei Elemente (63, 64) zur Festsetzung des Körpers (2) an dem Kopf (MT) des Mittelfußknochens erstrecken.

2. Implantat nach Anspruch 1, **dadurch gekennzeichnet, dass** die Festsetzungselemente (63, 64) sich von der konkaven Fläche (61) aus vorspringend in jeweilige Richtungen erstrecken, die im Wesentlichen parallel sind.

3. Implantat nach einem der Ansprüche 1 oder 2, **dadurch gekennzeichnet, dass** das (63) oder die Festsetzungselemente, die in einer dorsoplantaren Richtung der Plantarseite des Körpers (2) am nächsten sind, ein Vorsprungsmaß aufweisen, das am größten ist.

4. Implantat nach einem der vorhergehenden Ansprüche, **dadurch gekennzeichnet, dass** die zwei (63, 64) oder mindestens zwei der Festsetzungselemente in einer Sagittalmittelebene des Körpers (2) auf unterschiedliche Weise verteilt sind.

5. Implantat nach einem der vorhergehenden Ansprüche, **dadurch gekennzeichnet, dass** die zwei oder mindestens zwei der Festsetzungselemente gegenüber einer Sagittalmittelebene des Körpers (2) im Wesentlichen zueinander symmetrisch sind.

6. Implantat nach einem der vorhergehenden Ansprüche, **dadurch gekennzeichnet, dass** die konkave Fläche (61) eine im Wesentlichen kugelige Kappe umfasst oder sogar davon gebildet ist.

7. Implantat nach Anspruch 6, **dadurch gekennzeichnet, dass** die Tiefe (D) der Kappe (61) kleiner ist als 75 % des Radius (R) der geometrischen Kugel, zu der die Kappe gehört.

8. Implantat nach einem der vorhergehenden Ansprüche, **dadurch gekennzeichnet, dass** der Körper (2) an seiner Fläche (6), die zur Befestigung an dem Mittelfußknochen (M) bestimmt ist, einen knöchernen Abstützrand (62) begrenzt, der um die konkave Fläche (61) läuft, indem er gegebenenfalls im Wesentlichen in der geometrischen Ebene, welche die Kappe definiert, angeordnet ist.

9. Implantat nach einem der vorhergehenden Ansprüche, **dadurch gekennzeichnet, dass** die konvexe Fläche (41) eine mittlere Region (42) umfasst, die im Schnitt in einer Sagittalmittelebene des Körpers (2), einen Krümmungsradius (RS) aufweist, der kleiner ist als ihr Krümmungsradius (RH) im Schnitt in einer Horizontalmittelebene des Körpers.

10. Implantat nach einem der vorhergehenden Ansprüche, **dadurch gekennzeichnet, dass** der Körper (2) eine Umfangsfläche (8) begrenzt, die seine zwei Flächen (4, 6) verbindet, und eine im Wesentlichen kreisförmige Kontur (C8) aufweist, die durch ein Band (C81) mindestens auf der Plantarseite des Körpers unterbrochen ist.

11. Implantat nach einem der vorhergehenden Ansprüche, **dadurch gekennzeichnet, dass** der Körper (2) einstückig aus Keramik, insbesondere aus Pyrokohlenstoff hergestellt ist.

12. Implantat nach einem der Ansprüche 1 bis 10, **dadurch gekennzeichnet, dass** die Festsetzungsmittel (63, 64) mindestens teilweise aus einem Osteointegrationsmaterial bestehen.

## Claims

1. Hemiarthroplasty metatarsal implant (1),
comprising a distal and dorsal resurfacing body (2) for the articular head (MT) of a metatarsal (M), which body delimits:
- on its face (4) intended to articulate against the phalange (P) associated with the metatarsal, a convex surface (41) which is made of ceramic material, especially of pyrocarbon, and
- on its face (6) intended to be fixed to the metatarsal (M), a concave surface (61) from which there project out at least two elements (63, 64) for immobilising the body (2) on the head (MT) of the metatarsal.

2. Implant according to claim 1, **characterised in that** the immobilisation elements (63, 64) project out from the concave surface (61) in respective directions which are substantially parallel.

3. Implant according to one of claims 1 or 2, **characterised in that** the immobilisation element (63) or elements which, in a dorso-plantar direction, is/are closest to the plantar side of the body (2), has/have the greatest projection length.

4. Implant according to any one of the preceding claims, **characterised in that** the two (63, 64) or at least two of the immobilisation elements are differently distributed in a median sagittal plane of the body (2).

5. Implant according to any one of the preceding claims, **characterised in that** the two or at least two of the immobilisation elements are substantially symmetrical to one another relative to a median sagittal plane of the body (2).

6. Implant according to any one of the preceding claims, **characterised in that** the concave surface (61) comprises, or even consists of, a substantially spherical cap.

7. Implant according to claim 6, **characterised in that** the depth (D) of the cap (61) is less than 75 % of the radius (R) of the geometric sphere to which the cap belongs.

8. Implant according to any one of the preceding claims, **characterised in that** the body (2) delimits, on its face (6) intended to be fixed to the metatarsal (M), a bone abutment rim (62), which runs around the concave surface (61), optionally being arranged substantially in the geometric plane defining the cap.

9. Implant according to any one of the preceding claims, **characterised in that** the convex surface (41) includes a central region (42) which in a section in a median sagittal plane of the body (2) has a radius of curvature (RS) which is less than its radius of curvature (RH) in a section in a median horizontal plane of the body.

10. Implant according to any one of the preceding claims, **characterised in that** the body (2) delimits a peripheral surface (8) connecting its two faces (4, 6) which has a substantially circular contour (C8) interrupted by a chord (C81) at least on the plantar side of the body.

11. Implant according to any one of the preceding claims, **characterised in that** the body (2) is made of a single piece of ceramic material, especially of pyrocarbon.

12. Implant according to any one of claims 1 to 10, **characterised in that** the immobilisation elements (63, 64) are at least partly composed of an osseointegration material.
